# EUROPEAN PATENT SPECIFICATION

(11) **EP 3 910 074 B1**
(45) Date of publication and mention of the grant of the patent: **25.09.2024**
(21) Application number: 20738640.0
(22) Date of filing: 08.01.2020
(51) Int. Cl.: C12Q 1/6883, C12Q 1/6886, A23L 33/135, A61K 8/99, A61K 35/74, A61P 25/24, A61P 29/00, A61P 35/00

(54) **NANOVESICLES DERIVED FROM BACTERIA OF GENUS DEINOCOCCUS, AND USE THEREOF**
AUS BAKTERIEN DER GATTUNG DEINOCOCCUS GEWONNENE NANOVESIKEL UND VERWENDUNG DAVON
NANOVÉSICULES DÉRIVÉES DE BACTÉRIES DU GENRE PROPIONIBACTERIUM ET LEUR UTILISATION

(30) Priority: 09.01.2019 KR 20190002608; 07.01.2020 KR 20200002267
(43) Date of publication of application: 17.11.2021
(73) Proprietor: MD Healthcare Inc., Seoul 03923 (KR)
(72) Inventor: KIM, Yoon-Keun, Gyeonggi-do 10908 (KR)
(74) Representative: Dehns
(86) International application number: PCT/KR2020/000340
(87) International publication number: WO 2020/145661

(56) References cited:
- WO-A1-2016/153979
- WO-A1-2018/124617
- WO-A1-2020/252149
- KR-A- 20180 070 486
- KR-A- 20180 075 401
- KR-A- 20180 098 163
- KR-B1- 101 833 502
- US-B2- 7 628 995

## Description

### [Technical Field]

The present invention relates to a method of diagnosing stomach cancer or dementia using nanovesicles derived from bacteria of the genus *Deinococcus,* and a composition for use in preventing, alleviating or treating an inflammatory disease or a cranial nerve disease, which comprises vesicles derived from *Deinococcus radiodurans* as an active ingredient, wherein the inflammatory disease is a disease mediated by tumor necrosis factor-α (TNF-α), and wherein the cranial nerve disease is one or more selected from the group consisting of dementia, depression, Alzheimer's disease and autism.

### [Background Art]

Since the beginning of the 21st century, acute infectious diseases recognized as epidemic diseases in the past have become less important, whereas chronic inflammatory diseases accompanied by immune dysfunction caused by disharmony between humans and microbiomes have changed disease patterns as main diseases that determine the quality of life and the human lifespan. Cancer, cardiovascular diseases, chronic inflammatory diseases, metabolic diseases and neuro-psychiatric diseases, which are intractable chronic diseases in the 21st century, are major diseases that determine human lifetimes and the quality of life, and become a big challenge to public health.

It is known that the number of microorganisms coexisting in the human body has reached 100 trillion, which is 10 times more than the number of human cells, and the number of microorganism genes is more than 100 times the number of human genes. A microbiota or microbiome refers to a microbial community including bacteria, archaea and eukarya present in a given habitat.

Bacteria coexisting in our body and bacteria present in the ambient environment secrete nanometer-sized vesicles in order to exchange information on genes, low molecular compounds, proteins, and the like with other cells. The mucosa forms a physical defense membrane through which particles having a size of 200 nanometers (nm) or more cannot pass, so that bacteria coexisting in the mucosa cannot pass through the mucosa, but vesicles derived from bacteria have a size of 100 nanometers or less and are absorbed into our bodies after relatively freely passing through epithelial cells via the mucosa. Bacteria-derived vesicles that are locally secreted from bacteria are absorbed via epithelial cells of the mucous membrane to thereby induce a local inflammatory response, and the vesicles having passed through the epithelial cells are systematically absorbed via lymphatic vessels and thereby distributed in respective organs, and immune and inflammatory responses are regulated in the organs in which the vesicles are distributed. For example, vesicles derived from pathogenic gram-negative bacteria such as *Escherichia coli* locally cause inflammatory responses and cancer, and promote a systemic inflammatory response, and blood coagulation through a vascular endothelial inflammatory response when absorbed into blood vessels, and cause insulin resistance and diabetes when absorbed into cranial nerve cells and the like. On the other hand, vesicles derived from beneficial bacteria may control a disease by controlling immune dysfunction and metabolic dysfunction caused by pathogenic vesicles.

As immune responses to factors such as bacteria-derived vesicles, Th17 immune responses characterized by the secretion of the interleukin (hereinafter, IL)-17 cytokine occur, and IL-6 is secreted from epithelial cells, immune cells, and the like when exposed to bacteria-derived vesicles, thereby inducing Th17 immune responses characterized by the secretion of the IL-17. Inflammation caused by the Th17 immune response is characterized by neutrophil infiltration, and during the process by which inflammation occurs, tumor necrosis factor-alpha (hereinafter, TNF-α) secreted from inflammatory cells such as neutrocyte and macrophages plays an important role for occurrence of inflammation and cancer.

A brain-derived neurotrophic factor (BDNF) is a protein in the brain generated by BDNF gene, and one of a group of neurotrophic factors, as a part of the growth factors. The factor is associated with a basic nerve growth factor, and it is known that the expression of the factor is reduced in depression, dementia, Alzheimer's disease, autism, and the like.

Meanwhile, bacteria of the genus *Deinococcus* are bacteria that are the most resistant to radiation to date, and are known as bacteria that can survive even in a very difficult environment for living organisms, for example, cold, dehydration or strong acids. In the genus *Deinococcus,* to date, 47 species are known, and among these species, *Deinococcus radiodurans* is known as an absolute aerobic bacterium that is not pathogenic to humans, and a representative bacterium that survives even when exposed to radiation. However, so far, there have been no cases of application of vesicles derived from bacteria of the genus *Deinococcus* in diagnosis and treatment of intractable diseases such as cancer, inflammatory diseases and dementia.

KR 2018 0075401 discloses a method for diagnosis of bladder cancer using analysis of microbial metagenome.

WO 2020/252149 discloses methods and pharmaceutical compositions related to processed microbial extracellular vesicles (pmEVs) that can be useful as therapeutic agents.

KR 2018 0098163 discloses a method for diagnosis of chronic obstructive airway disease using analysis of bacteria metagenome.

Accordingly, in the present invention, it was confirmed that vesicles derived from bacteria of the genus *Deinococcus* significantly decrease in clinical samples of stomach cancer patients and dementia patients, compared to a normal individual, thereby diagnosing the diseases. In addition, as a result of isolating vesicles from *Deinococcus radiodurans* belonging to the genus *Deinococcus* and evaluating the therapeutic effect thereof, it was confirmed that they can be used as a composition for use in preventing, treating, or alleviating an inflammatory disease and a cranial nerve disease, wherein the inflammatory disease is a disease mediated by tumor necrosis factor-α (TNF-α), and wherein the cranial nerve disease is one or more selected from the group consisting of dementia, depression, Alzheimer's disease and autism.

### [Disclosure]

### [Technical Problem]

As a result of conducting earnest research to solve the above conventional problems, the inventors confirmed that a content of vesicles derived from bacteria of the genus *Deinococcus* is significantly decreased in a sample derived from a patient with stomach cancer or dementia, compared with a normal individual, through metagenomic analysis. In addition, when macrophages were treated with vesicles isolated from *Deinococcus radiodurans* belonging to the genus *Deinococcus,* it was confirmed that TNF-α secretion mediated by pathogenic vesicles is remarkably inhibited, and damage to cranial nerve cells caused by a stress hormone was significantly inhibited.

Thus, an object of the present invention is to provide a method of diagnosing stomach cancer or dementia.

Further, another object of the present invention is to provide a composition for use in preventing, alleviating, or treating one or more diseases selected from the group consisting of an inflammatory disease, and a cranial nerve disease, comprising vesicles derived from *Deinococcus radiodurans* as an active ingredient, and wherein the inflammatory disease is a disease mediated by tumor necrosis factor-α (TNF-α), and wherein the cranial nerve disease is one or more selected from the group consisting of dementia, depression, Alzheimer's disease and autism.

However, a technical problem to be achieved by the present invention is not limited to the aforementioned problems, and the other problems that are not mentioned may be clearly understood by a person skilled in the art from the following description.

### [Technical Solution]

To achieve the object of the present invention as described above, the present invention provides a composition for use in preventing, treating, or alleviating one or more diseases selected from the group consisting of an inflammatory disease, and a cranial nerve disease, and a method of diagnosing stomach cancer or dementia, as defined in the appended claims.

The present invention provides a method of diagnosing stomach cancer or dementia, the method comprising the following steps:
(a) extracting DNAs from vesicles isolated from samples of a normal individual and a subj ect;
(b) performing PCR on the extracted DNA using a pair of primers prepared based on a gene sequence present in 16S rDNA to obtain each PCR product; and
(c) determining a case in which a content of vesicles derived from bacteria of the genus *Deinococcus* is lower than that of the normal individual sample, as stomach cancer or dementia, through quantitative analysis of the PCR product.

As an embodiment of the present invention, the sample in Step (a) is not limited, but may be blood, urine, stool, saliva or nasal mucosa, and preferably blood.

As another embodiment of the present invention, the primer pair in Step (b) may be primers of SEQ ID Nos. 1 and 2.

Further, the present invention provides a composition for use in preventing, treating, or alleviating one or more diseases selected from the group consisting of an inflammatory disease, and a cranial nerve disease, comprising vesicles derived from *Deinococcus radiodurans* as an active ingredient, and wherein the inflammatory disease is a disease mediated by tumor necrosis factor-α (TNF-α), and wherein the cranial nerve disease is one or more selected from the group consisting of dementia, depression, Alzheimer's disease and autism.

As another embodiment of the present invention, the inflammatory disease may be one or more selected from the group consisting of gingivitis, periodontitis, gastritis, inflammatory enteritis, colitis, atopic dermatitis, acne, hair loss, psoriasis, rhinitis, nasal polyps, asthma, chronic obstructive pulmonary disease (COPD), degenerative arthritis, and rheumatoid arthritis. As an embodiment of the present invention, the vesicles may have an average diameter of 10 to 200 nm.

As another embodiment of the present invention, the vesicles may be secreted naturally or artificially from *Deinococcus radiodurans.*

As another embodiment of the present invention, the composition may be a pharmaceutical composition, a food composition, an inhalant composition, or a cosmetic composition.

### [Advantageous Effects]

The inventors confirmed that bacteria are not absorbed into the body through epithelial cells, but bacteria-derived vesicles are absorbed, systemically distributed and then excreted out of the body through the kidneys, liver and lungs, and by metagenomic analysis for vesicles derived from bacteria present in patients' blood, also confirmed that vesicles derived from bacteria of the genus *Deinococcus,* which are present in blood of patients with cancer, an inflammatory disease, and dementia, significantly decrease, compared with a normal individual. In addition, when vesicles were isolated by culturing *Deinococcus radiodurans*, which is one bacterial species of the genus *Deinococcus* in vitro, and administered into inflammatory cells in vitro, not only was it observed that the secretion of an inflammation mediator by pathogenic vesicles is significantly inhibited, but it was also confirmed that the vesicles derived from *Deinococcus radiodurans* significantly inhibits damage to cranial nerve cells by a stress hormone.

Therefore, the vesicles derived from bacteria of the genus *Deinococcus* are expected to be effectively used in a method of diagnosing stomach cancer or dementia. The vesicles derived from *Deinococcus radiodurans* are also expected to be effectively used in a composition for use in preventing, alleviating or treating an inflammatory disease or a cranial nerve disease, wherein the inflammatory disease is a disease mediated by tumor necrosis factor-α (TNF-α), and wherein the cranial nerve disease is one or more selected from the group consisting of dementia, depression, Alzheimer's disease and autism, for example, cosmetics, food, inhalants or drugs.

### [Description of Drawings]

FIG. 1A is a series of photographs capturing distribution patterns of bacteria and bacteria-derived vesicles (EV) by time after the bacteria and the vesicles derived from bacteria were orally administered to mice, and FIG. 1B is a result of evaluating the in vivo distribution patterns of the bacteria and the vesicles by harvesting blood, kidneys, liver, and various organs at 12 hours after orally administering the bacteria and the vesicles.
FIG. 2 shows images of evaluating the infiltration of bacteria and bacteria-derived vesicles into intestinal mucosa epithelial cells after bacteria and bacteria-derived vesicles (EV) are injected into the mouse intestines (Lu: gut lumen; LP: gut lamina propria).
FIG. 3 is a result of comparing the distributions of vesicles derived from bacteria of the genus *Deinococcus* after metagenomic analysis of bacteria-derived vesicles present in the blood of stomach cancer patients and a normal individual.
FIG. 4 is a result of comparing the distributions of vesicles derived from bacteria of the genus *Deinococcus* after metagenomic analysis of bacteria-derived vesicles present in the blood of asthma patients and a normal individual.
FIG. 5 is a result of comparing the distributions of vesicles derived from bacteria of the genus *Deinococcus* after metagenomic analysis of bacteria-derived vesicles present in the blood of dementia patients and a normal individual.
FIG. 6 is a result of evaluating an effect on the secretion of inflammatory mediators (TNF-α) caused by *E. coli* EVs by pretreating vesicles derived from *Deinococcus radiodurans* before treatment of pathogenic vesicles such as *E. coli* EVs to evaluate anti-inflammatory effects of *Deinococcus radiodurans-derived* vesicles (EV: extracellular vesicle).
FIG. 7 shows the result of evaluating the influence on brain-derived neurotrophic factor (BDNF) expression by nerve cells by simultaneously treating nerve cells, which have been treated with an adrenocortical hormone (GC), which is a stress hormone, with *Deinococcus radiodurans-derived* vesicles to evaluate a nerve cell protective effect of the *Deinococcus radiodurans-derived* vesicles (EV: *Deinococcus radiodurans* extracellular vesicle).

### [Best Modes]

The present invention relates to vesicles derived from bacteria of the genus *Deinococcus* and a use thereof.

The inventors confirmed that a content of vesicles derived from bacteria of the genus *Deinococcus* are significantly reduced in samples of patients with cancer, an inflammatory disease, and dementia, compared with a normal individual, through metagenomic analysis.

Thus, the present invention provides a method of diagnosing stomach cancer or dementia, the method comprising the following steps:
(a) extracting DNAs from vesicles isolated from samples of a normal individual and a subj ect;
(b) performing PCR on the extracted DNA using a pair of primers prepared based on a gene sequence present in 16S rDNA to obtain each PCR product; and
(c) determining a case in which a content of vesicles derived from bacteria of the genus *Deinococcus* is lower than that of the normal individual sample, as stomach cancer or dementia, through quantitative analysis of the PCR product.

The term "diagnosis" as used herein refers to determination of a condition of a disease of a patient over all aspects, in a broad sense. The contents of the determination are the disease entity, the etiology, the pathogenesis, the severity, the detailed aspects of a disease, the presence and absence of complications, the prognosis, and the like. The diagnosis in the present invention means determining whether stomach cancer and/or dementia occur, the level of the disease, and the like.

The term "nanovesicle" or "vesicle" as used herein refers to a structure consisting of a nano-sized membrane secreted from various bacteria. Vesicles derived from gram-negative bacteria or outer membrane vesicles (OMVs) have endotoxins (lipopolysaccharides) or glycosphingolipid, toxic protein, and bacterial DNA and RNA, and vesicles derived from gram-positive bacteria also have peptidoglycan and lipoteichoic acid which are cell wall components of bacteria in addition to proteins and nucleic acids. In the present invention, nanovesicles or vesicles are secreted naturally from bacteria of the genus *Deinococcus* or produced artificially, are in the form of a sphere, and have an average diameter of 10 to 200 nm.

The term "metagenome" as used herein also refers to a microbiome, and refers to a total of genomes including all viruses, bacteria, fungi, and the like in an isolated region such as soil and an animal's intestines, and is typically used as a concept of genomes explaining identification of a large number of microorganisms at one time by using a sequence analyzer in order to analyze uncultivated microorganisms. In particular, the metagenome does not refer to a genome of one species, but refers to a kind of mixed genome as a genome of all species of one environmental unit. The metagenome is, when one species is defined in the development process of omics biology, a term derived from the viewpoint of making a complete species is made by various species interacting with each other as well as one kind of functionally existing species. Technically, the metagenome is an object of a technique to identify all species in one environment and investigate interactions and metabolism by analyzing all DNAs and RNAs regardless of species using a rapid sequence analysis method.

The vesicles may be isolated from a culturing solution comprising bacteria of the genus *Deinococcus* by using one or more methods selected from the group consisting of centrifugation, ultra-high speed centrifugation, high pressure treatment, extrusion, sonication, cell lysis, homogenization, freezing-thawing, electroporation, mechanical decomposition, chemical treatment, filtration by a filter, gel filtration chromatography, free-flow electrophoresis, and capillary electrophoresis. Further, a process such as washing for removing impurities and concentration of obtained vesicles may be further included.

In the present invention, the sample in Step (a) is not limited, but may be blood, urine, stool, saliva or nasal mucosa, and preferably blood.

In the present invention, the primer pair in Step (b) may be primers of SEQ ID Nos. 1 and 2, but is not limited thereto.

Another aspect of the present invention provides a composition for use in preventing, treating, or alleviating one or more diseases selected from the group consisting of an inflammatory disease, and a cranial nerve disease, comprising vesicles derived from *Deinococcus radiodurans* as an active ingredient, wherein the inflammatory disease is a disease mediated by tumor necrosis factor-α (TNF-α), and wherein the cranial nerve disease is one or more selected from the group consisting of dementia, depression, Alzheimer's disease and autism.

In the present invention, the composition includes a pharmaceutical composition, a food composition, an inhalant composition, and a cosmetic composition.

The term "prevention" as used herein refers to all actions that suppress an inflammatory disease and/or a cranial nerve disease, as defined elsewhere herein, or delay the onset thereof via administration of the composition according to the present invention.

The term "treatment" as used herein refers to all actions that alleviate or beneficially change symptoms of an inflammatory disease and/or a cranial nerve disease, as defined elsewhere herein, or the like via administration of composition according to the present invention.

The term "alleviation" used as used herein refers to all actions that at least reduce a parameter associated with a condition to be treated, for example, the degree of symptoms.

The term "subject" used herein refers to a target in need of treatment of cancer, an inflammatory disease or a cranial nerve disease, as defined elsewhere herein, and more specifically, a mammal such as a human or a non-human primate, a mouse, a dog, a cat, a horse, a cow, and the like.

The term "administration" used herein means providing a composition for use of the present invention to a subject by any suitable method.

The term "cancer" used herein refers to a disease that forms a mass or tumor consisting of undifferentiated cells neglecting the order in tissue and unlimitedly proliferating, and ultimately, the generic term for a group of diseases that form a novel growth site by disrupting normal peripheral tissue or organs through infiltration and inducing metastasis from the primary lesion to any organ of a subject. In the present invention, the cancer is stomach cancer.

The term "inflammatory disease" used herein refers to a disease caused by a chain of biological reactions occurring by a direct reaction of a humoral mediator constituting the immune system or stimulation of a local or systemic effector system, and in the present invention, the inflammatory disease may be one or more selected from the group consisting of gingivitis, periodontitis, gastritis, inflammatory enteritis, colitis, atopic dermatitis, acne, hair loss, psoriasis, rhinitis, nasal polyps, asthma, chronic obstructive pulmonary disease (COPD), degenerative arthritis, and rheumatoid arthritis, but the present invention is not limited thereto.

In the present invention, the inflammatory disease is a disease mediated by tumor necrosis factor-α (TNF-α).The term "cranial nerve disease" used herein is a generic term for diseases caused by problems with cranial nerve cells, and in the present invention, the cranial nerve disease is one or more selected from the group consisting of depression, dementia, Alzheimer's disease and autism.

The pharmaceutical composition for use of the present invention may include a pharmaceutically acceptable carrier. The pharmaceutically acceptable carrier is typically used in formulation, and includes saline, sterile water, Ringer's solution, buffered saline, cyclodextrin, a dextrose solution, a maltodextrin solution, glycerol, ethanol, liposomes, and the like, but is not limited thereto, and may further include other typical additives such as an antioxidant and a buffer, if necessary. Further, the composition may be formulated into an injectable formulation, such as an aqueous solution, a suspension, and an emulsion, a pill, a capsule, a granule, or a tablet by additionally adding a diluent, a dispersant, a surfactant, a binder, a lubricant, and the like. With regard to suitable pharmaceutically acceptable carriers and formulations, the composition may be preferably formulated according to each ingredient by using the method disclosed in the Remington's literature. The pharmaceutical composition for use of the present invention is not particularly limited in formulation, but may be formulated into an injection, an inhalant, an external preparation for skin, an oral ingestion, or the like.

The pharmaceutical composition for use of the present invention may be administered orally or parenterally (e.g., intravenously, subcutaneously, intradermally) according to a desired method, and a dose may vary according to the condition and body weight of a patient, the severity of a disease, a drug formulation, an administration route, and duration, but may be suitably selected by those of ordinary skill in the art.

The pharmaceutical composition for use according to the present invention is administered in a pharmaceutically effective amount. In the present invention, the pharmaceutically effective amount refers to an amount sufficient to treat diseases at a reasonable benefit/risk ratio applicable to medical treatment, and an effective dosage level may be determined according to factors including types of diseases of patients, the severity of disease, the activity of drugs, sensitivity to drugs, administration time, administration route, excretion rate, treatment period, and simultaneously used drugs, and factors well known in other medical fields. The composition for use according to the present invention may be administered as an individual therapeutic agent or in combination with other therapeutic agents, may be administered sequentially or simultaneously with therapeutic agents in the related art, and may be administered in a single dose or multiple doses. It is important to administer the composition in a minimum amount that can obtain the maximum effect without any side effects, in consideration of all the aforementioned factors, and this may be easily determined by those of ordinary skill in the art.

Specifically, the effective amount of the pharmaceutical composition for use according to the present invention may be changed according to a patient's age, sex or body weight, and may be increased or decreased depending on the route of administration, the severity of obesity, sex, a body weight, age or the like.

The food composition for use of the present invention includes a health functional food composition. The food composition for use according to the present invention may be used by adding an active ingredient as is to food or may be used together with other foods or food ingredients, but may be appropriately used according to a typical method. The mixed amount of the active ingredient may be suitably determined depending on the purpose of use thereof (for prevention or alleviation). In general, when a food or beverage is prepared, the composition for use of the present invention is added in an amount of 15 wt% or less, preferably 10 wt% or less based on the raw materials. However, for long-term intake for the purpose of health and hygiene or for the purpose of health control, the amount may be less than the above-mentioned range.

Other ingredients are not particularly limited, except that the food composition for use of the present invention contains the active ingredient as an essential ingredient at the indicated ratio, and the food composition for use of the present invention may contain various flavorants, natural carbohydrates, and the like, like a typical beverage, as an additional ingredient. Examples of the above-described natural carbohydrate include common sugars such as monosaccharides, for example, glucose, fructose and the like; disaccharides, for example, maltose, sucrose and the like; and polysaccharides, for example, dextrin, cyclodextrin and the like, and sugar alcohols such as xylitol, sorbitol, and erythritol. As the flavorant other than those described above, a natural flavorant (thaumatin, stevia extract, for example, rebaudioside A, glycyrrhizin and the like), and a synthetic flavorant (saccharin, aspartame and the like) may be advantageously used. The proportion of the natural carbohydrate may be appropriately determined by the choice of those of ordinary skill in the art.

The food composition for use of the present invention may contain various nutrients, vitamins, minerals (electrolytes), flavoring agents such as synthetic flavoring agents and natural flavoring agents, colorants and fillers (cheese, chocolate, and the like), pectic acid and salts thereof, alginic acid and salts thereof, organic acids, protective colloid thickeners, pH adjusting agents, stabilizers, preservatives, glycerin, alcohols, carbonating agents used in a carbonated beverage, or the like, in addition to the additives. These ingredients may be used either alone or in combinations thereof. The ratio of these additives may also be appropriately selected by those of ordinary skill in the art.

The inhalant composition for use of the present invention may include not only vesicles derived from *Deinococcus radiodurans*, but also components conventionally used in inhalant compositions, for example, conventional adjuvants such as an antioxidant, a stabilizer, a solubilizer, a vitamin and a flavoring agent, and a carrier.

The cosmetic composition for use of the present invention may comprise ingredients conventionally used in a cosmetic composition as well as vesicles derived from *Deinococcus radiodurans,* and may comprise, for example, a conventional additive such as an antioxidant, a stabilizer, a solubilizer, a vitamin, a pigment and a flavor, and a carrier.

In addition, the composition for use of the present invention may also be used by mixing a conventionally used organic sunscreen as long as it does not impair a skin protection effect by reaction with the vesicles derived from *Deinococcus radiodurans*, in addition to the vesicles derived from *Deinococcus radiodurans.* The organic sunscreen may be one or more selected from the group consisting of glyceryl PABA, drometrizole trisiloxane, drometrizole, digalloyl trioleate, disodium phenyl dibenzimidazole tetrasulfonate, diethylhexyl butamidotriazone, diethylamino hydroxybenzoyl hexylbenzoate, DEA-methoxycinnamate, a Lawson/dihydroxyacetone mixture, methylenebis-benzotriazolyltetramethylbutylphenol, 4-methylbenzylidene camphor, methyl anthranilate, benzophenone-3(oxybenzone), benzophenone-4, benzophenone-8(dioxyphebenzone), butyl methoxydibenzoylmethane, bisethylhexyloxyphenol methoxyphenyl triazine, cinoxate, ethyl dihydroxypropyl PABA, octocrylene, ethylhexyldimethyl PABA, ethylhexyl methoxycinnamate, ethylhexyl salicylate, ethylhexyl triazone, isoamyl-p-methoxycinnamate, polysilicon-15 (dimethicodiethylbenzal malonate), terephthalylidene dicamphor sulfonic acid and a salt thereof, TEA-salicylate and aminobenzoic acid (PABA).

Products that can contain the cosmetic composition for use of the present invention include, for example, cosmetics such as an astringent, a skin toner, a nourishing toner, various types of creams, essences, packs and foundations, cleansers, face washes, soaps, treatments, and tonics. Specific formulations of the cosmetic composition for use of the present invention include a skin lotion, a skin softener, a skin toner, an astringent, a lotion, a milk lotion, a moisturizing lotion, a nourishing lotion, a massage cream, a nourishing cream, a moisturizing cream, a hand cream, an essence, a nourishing essence, a pack, a soap, a shampoo, a cleansing foam, a cleansing lotion, a cleansing cream, a body lotion, a body cleanser, an emulsion, a lipstick, a makeup base, a foundation, a pressed powder, a loose powder, and an eyeshadow.

As a result of orally administering bacteria and bacteria-derived vesicles to mice and observing in vivo absorption, distribution, and excretion patterns of the bacteria and the vesicles, it was confirmed that, while the bacteria were not absorbed via the intestinal mucous membrane, the bacteria-derived vesicles were absorbed within 5 minutes after administration and systemically distributed, and excreted via the kidneys, liver, and the like (see Example 1).

It was evaluated whether bacteria and vesicles derived from bacteria directly administered to the intestines passed through the protective membrane of the intestinal mucosa, and it was confirmed that bacteria failed to pass through the protective membrane of the intestinal mucosa, whereas vesicles derived from bacteria passed through the protective membrane of the mucosa. (See Example 2).

A bacterial metagenomic analysis was performed by using vesicles isolated from the blood of normal individuals who were matched in age and sex with patients with cancer, an inflammatory disease, and dementia. As a result, it was confirmed that vesicles derived from bacteria of the genus *Deinococcus* were significantly decreased in clinical samples of patients with cancer, an inflammatory disease, and dementia as compared to samples of normal individuals (see Examples 4, 5, and 6).

Anti-inflammatory effects of vesicles derived from *Deinococcus radiodurans* strains were evaluated. As a result of evaluating the secretion of inflammatory mediators after treating macrophages with vesicles derived from *Deinococcus radiodurans* at various concentrations prior to treatment with *E*. *coli*-derived vesicles, which are pathogenic vesicles, it was confirmed the vesicles derived from *Deinococcus radiodurans* efficiently suppressed the secretion of TNF-α, which is a mediator inducing inflammation and cancer, mediated by inflammation-inducing *E*. *coli*-derived vesicles (see Example 8).

The nerve cell protective effect of vesicles derived from a *Deinococcus radiodurans* strain was evaluated, and as a result of evaluating brain-derived neurotrophic factor (BDNF) expression by simultaneously treating nerve cells, which have been treated with an adrenocortical hormone, which stresses nerve cells, with *Deinococcus radiodurans-derived* vesicles, it was confirmed that the *Deinococcus radiodurans-derived* vesicles effectively increase the expression of BDNF, which is a mediator protecting against nerve cell damage (see Example 9).

Hereinafter, preferred Examples for helping the understanding of the present invention will be suggested. However, the following Examples are provided only to more easily understand the present invention, and the contents of the present invention are not limited by the following Examples.

### [Examples]

### Example 1. Analysis of in vivo Absorption, Distribution, and Excretion Patterns of Bacteria and Vesicles Derived from Bacteria

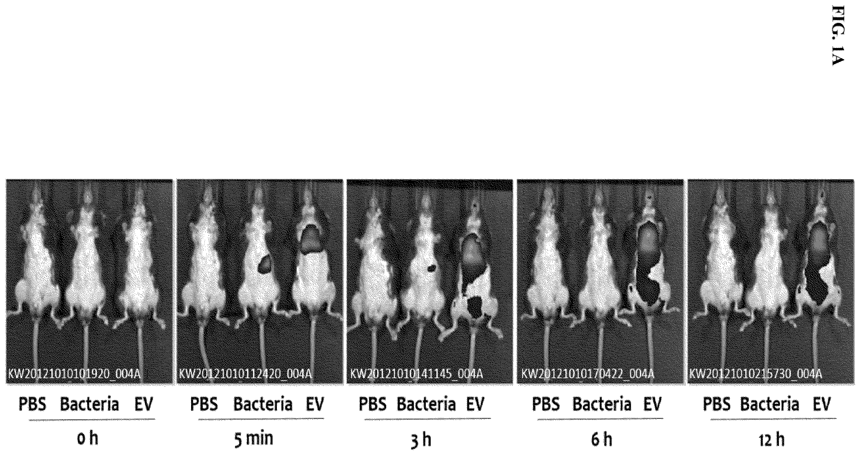

In order to evaluate whether bacteria and bacteria-derived vesicles were systemically absorbed through the gastrointestinal tract, an experiment was performed with the following method. First, a dose of 50 µg of each of fluorescence-labeled bacteria and the bacteria-derived vesicles was administered through the gastrointestinal tract to the stomach of a mouse, and fluorescence was measured after 0 minute, 5 minutes, 3 hours, 6 hours, and 12 hours. As a result of observing the entire image of the mouse, as illustrated in FIG. 1A, the bacteria were not systemically absorbed, but the vesicles derived from bacteria were systemically absorbed 5 minutes after administration, and fluorescence was strongly observed in the bladder 3 hours after administration, so that it could be seen that the vesicles were excreted to the urinary tract. Further, it could be seen that the vesicles were present in the body until 12 hours after administration (see FIG. 1A).

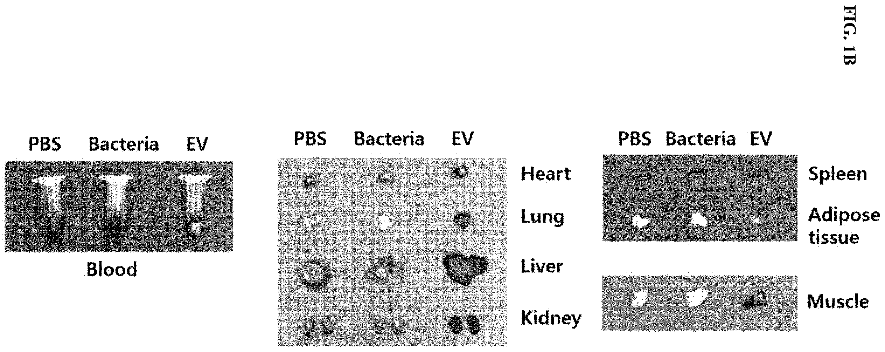

In addition, in order to evaluate the pattern in which the intestinal bacteria and the vesicles derived from the intestinal bacteria infiltrated into various organs after they were systemically absorbed, 50 µg of bacteria and vesicles derived from bacteria labeled with fluorescence were administered in the same manner as described above, and then the urine, heart, lungs, liver, kidneys, spleen, fat, and muscle were collected 12 hours after administration. As a result of observing fluorescence in the blood and collected tissues, as illustrated in FIG. 1B, it could be seen that the vesicles derived from bacteria were distributed in the urine, heart, lungs, liver, spleen, fat, muscle, and kidneys but the bacteria were not absorbed (see FIG. 1B).

### Example 2. Evaluation of whether bacteria and vesicles derived from bacteria penetrate protective membrane of intestinal mucosa

In order to evaluate whether bacteria and bacteria-derived vesicles passed through the protective membrane of the mucosa to be infiltrated into tissue, after bacteria and bacteria-derived vesicles were directly administered to the intestines, infiltration into the intestinal tissue after passing through the protective membrane of the mucosa was evaluated by an immunohistochemistry method. In order to evaluate the presence of bacteria and vesicles in the mucosa tissue, antibodies against the bacteria and the vesicles were prepared, attached to a green fluorescent protein (GFP) and used, and after staining with 4, 6-diamidino 2-phenylindole (DAPI), observed under a microscope.

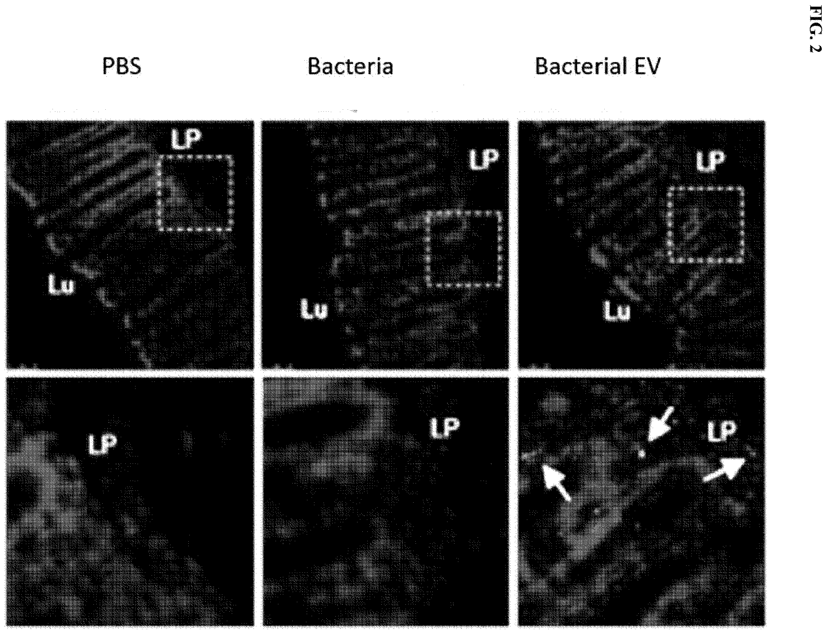

As a result, it was confirmed that the bacteria cannot pass through the protective membrane of the mucosal, but the bacteria-derived vesicles infiltrate into the intestinal tissue through the mucosa (see FIG. 2).

### Example 3. Metagenomic Analysis of Vesicles Derived from Bacteria in Clinical Sample

After blood was first put into a 10-ml tube and suspended matter was allowed to settle by a centrifuge (3,500 x g, 10 min, 4°C), only the supernatant was transferred to a new 10-ml tube. After bacteria and impurities were removed by using a 0.22-µm filter, they were transferred to a Centriprep tube (centrifugal filters 50 kD) and centrifuged at 1,500 x g and 4°C for 15 minutes, materials smaller than 50 kD were discarded, and the residue was concentrated to 10 ml. After bacteria and impurities were removed once again by using a 0.22-µm filter, the supernatant was discarded by using a ultra-high speed centrifugation at 150,000 x g and 4°C for 3 hours with a Type 90Ti rotor, and an aggregated pellet was dissolved in physiological saline (PBS).

Internal DNA was extracted out of the lipid by boiling 100 µl of the vesicles isolated by the above method at 100°C, and then cooled on ice for 5 minutes. And then, in order to remove the remaining suspended matter, the DNA was centrifuged at 10,000 x g and 4°C for 30 minutes, and only the supernatant was collected. And, the amount of DNA was quantified by using Nanodrop. Thereafter, in order to confirm whether the DNA derived from bacteria was present in the extracted DNA, PCR was performed with 16s rDNA primers shown in the following Table 1 and it was confirmed that genes derived from bacteria were present in the extracted genes.

**[Table 1]**

| primer | | Sequence | SEQ ID No. |
|---|---|---|---|
| 16S rDNA | 16S_V3_F | 5'- TCGTCGGCAGCGTCAGATGTGTATAAGA GACAGCCTACGGGNGGCWGCAG-3' | 1 |
| | 16S_V4_R | 5'- GTCTCGTGGGCTCGGAGATGTGTATAAG AGACAGGACTACHVGGGTATCTAATCC | 2 |

The DNA extracted by the above method was amplified using the 16S rDNA primers, and then sequencing was performed (Illumina MiSeq sequencer), the results were output as a standard flowgram format (SFF) file, the SFF file was converted into a sequence file (.fasta) and a nucleotide quality score file using GS FLX software (v2.9), and then the reliability estimation for the reads was confirmed, and a portion in which the window (20 bps) average base call accuracy was less than 99% (Phred score<20) was removed. For the OTU (operational taxonomy unit) analysis, clustering was performed according to sequence similarity by using UCLUST and USEARCH, the genus, family, order, class, and phylum were clustered based on 94%, 90%, 85%, 80%, and 75% sequence similarity, respectively, classification was performed at the phylum, class, order, family, and genus levels of each OUT, and bacteria having a sequence similarity of 97% or more at the genus level were profiled by using the 16S RNA sequence database (108,453 sequences) of BLASTN and GreenGenes (QIIME).

### Example 4. Metagenomic analysis of bacteria-derived vesicles in blood of patient with gastric cancer

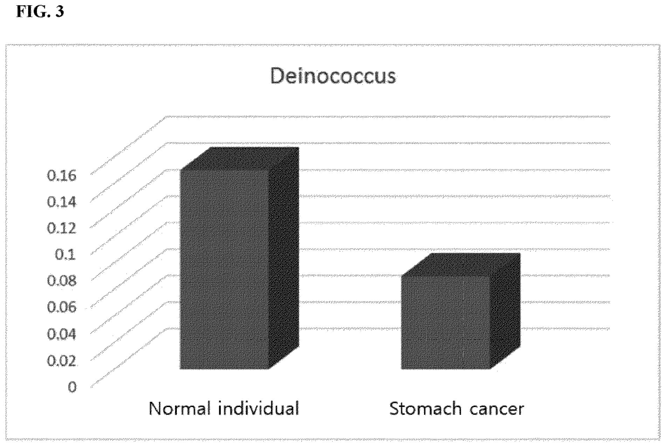

After a metagenomic analysis was performed using the method of Example 3 on the blood from 87 patients with gastric cancer, and 91 normal individuals who were matched in age and sex by extracting genes from vesicles present in the blood, the distribution of vesicles derived from bacteria of the genus *Deinococcus* was evaluated. As a result, it was confirmed that vesicles derived from bacteria of the genus *Deinococcus* were significantly decreased in the blood from the patients with gastric cancer as compared to the blood from the normal individuals (see FIG. 3).

### Example 5. Metagenomic analysis of bacteria-derived vesicles in blood of patient with asthma

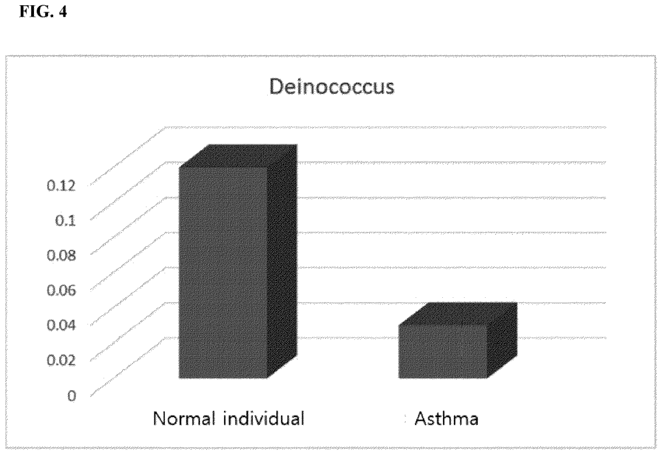

After a metagenomic analysis was performed using the method of Example 3 on the blood from 132 patients with asthma, and 109 normal individuals who were matched in age and sex by extracting genes from vesicles present in the blood, the distribution of vesicles derived from bacteria of the genus *Deinococcus* was evaluated. As a result, it was confirmed that vesicles derived from bacteria of the genus *Deinococcus* were significantly decreased in the blood from the patients with asthma as compared to the blood from the normal individuals (see FIG. 4).

### Example 6. Metagenomic analysis of bacteria-derived vesicles in blood of patient with dementia

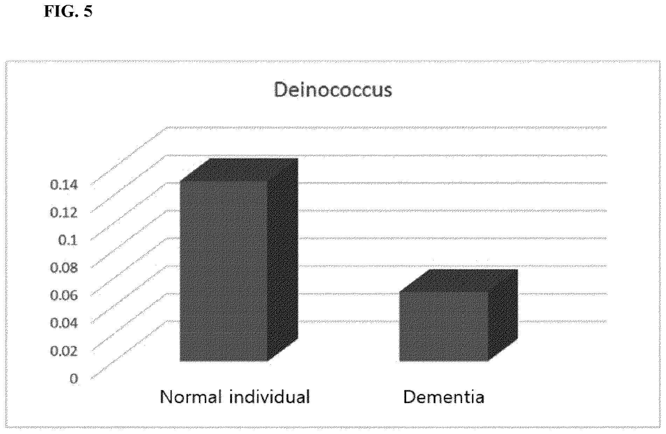

After a metagenomic analysis was performed using the method of Example 3 on the blood from 82 patients with dementia, and 99 normal individuals who were matched in age and sex by extracting genes from vesicles present in the blood, the distribution of vesicles derived from bacteria of the genus *Deinococcus* was evaluated. As a result, it was confirmed that vesicles derived from bacteria of the genus *Deinococcus* were significantly decreased in the blood from the patients with dementia as compared to the blood from the normal individuals (see FIG. 5).

### Example 7. Isolation of Vesicles from Deinococcus radiodurans Culturing Solution

Based on the above examples, a *Deinococcus radiodurans* strain were cultured, and then vesicles were isolated therefrom and characteristics of the isolated vesicles were analyzed. First, the *Deinococcus radiodurans* strains were cultured in a de Man-Rogosa and Sharpe (MRS) medium in an incubator at 37°C until the absorbance (OD 600) became 1.0 to 1.5, and then sub-cultured in a Luria-Bertani (LB) medium. Subsequently, a culture supernatant including the strain was recovered and centrifuged at 10,000 x g and 4 °C for 20 minutes, and then the strain was removed and filtered through a 0.22 µm filter. The filtered supernatant was concentrated to a volume of less than or equal to 50 ml through microfiltration by using a MasterFlex pump system (Cole-Parmer, US) with a 100 kDa Pellicon 2 Cassette filter membrane (Merck Millipore, US), and the concentrated supernatant was filtered once again with a 0.22-µm filter. Thereafter, proteins were quantified by using a BCA assay, and the following experiments were performed on the obtained vesicles.

### Example 8. Anti-inflammatory Effects of Vesicles derived from Deinococcus radiodurans

In order to evaluate the anti-inflammatory effects of vesicles derived from *Deinococcus radiodurans*, after mouse macrophage cell lines were pre-treated with vesicles derived from *Deinococcus radiodurans* (D. radiodurans EV) at various concentrations (0.1, 1, and 10 µg/ml) for 12 hours, the cell lines were treated with 1 µg/ml of *E*. *coli*-derived vesicles, which are a pathogenic factor, and then the secretion of inflammatory cytokines was measured by ELISA after 12 hours.

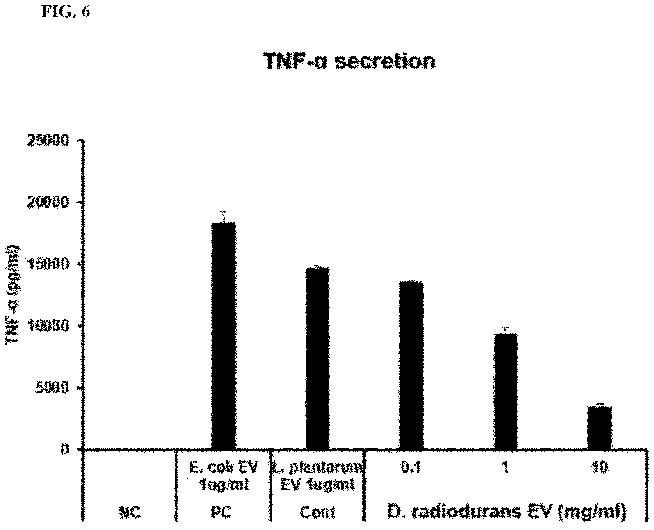

As a result, upon pre-treatment of the *Deinococcus radiodurans-derived* vesicles, it was confirmed that the secretion of TNF-α, which is an inflammation mediator closely associated with cell death, inflammation and cancer induced in inflammatory cells by *E. coli-*derived vesicle stimulation, is inhibited by *Deinococcus radiodurans-derived* vesicles in a dose-dependent manner (see FIG. 6).

### Example 9. Nerve cell protective effect of Deinococcus radiodurans-derived vesicles

A brain-derived neurotrophic factor (BDNF) is a major mediator protecting nerve cells from nerve cell damage, and its expression is decreased in dementia, depression, Alzheimer's disease, autism, and the like. In this example, to evaluate the therapeutic effect of *Deinococcus radiodurans-derived* vesicles against a cranial nerve disease, the nerve cell protective effect was evaluated by treating the nerve cells with a stress hormone. That is, nerve cells (hippocampal neuronal cell line, HT22 cells) were cultured with an adrenocortical hormone (GC: corticosterone 400 ng/ml) or *Deinococcus radiodurans-derived* vesicles (EV, 20 µg/mL) for 24 hours in vitro, and the BDNF expression was evaluated by PCR.

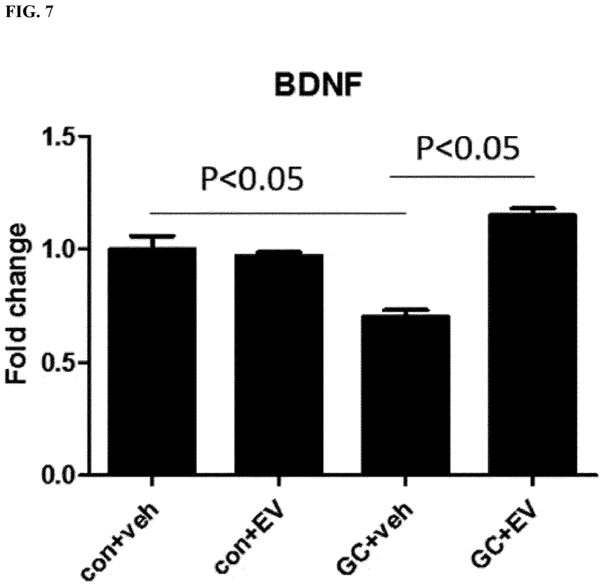

As a result, it was confirmed that the BDNF expression inhibited by adrenocortical hormone treatment is significantly recovered by the treatment of *Deinococcus radiodurans-*derived vesicles (see FIG. 7). This means that the *Deinococcus radiodurans-derived* vesicles can effectively inhibit a cranial nerve disease caused by a factor causing cranial nerve cell damage such as stress.

It should be understood that the above-described Examples are illustrative only in all aspects and are not restrictive.

## Claims

1. A composition for use in preventing, treating, or alleviating one or more diseases selected from the group consisting of an inflammatory disease and a cranial nerve disease, comprising vesicles derived from *Deinococcus radiodurans* as an active ingredient, and
wherein the inflammatory disease is a disease mediated by tumor necrosis factor-α (TNF-α), and
wherein the cranial nerve disease is one or more selected from the group consisting of dementia, depression, Alzheimer's disease and autism.

2. The composition for use of claim 1, wherein the vesicles have an average diameter of 10 to 200 nm.

3. The composition for use of claim 1 or claim 2, wherein the vesicles are secreted naturally or artificially from *Deinococcus radiodurans.*

4. The composition for use of any one of claims 1 to 3, wherein the composition is a pharmaceutical composition, a food composition, an inhalant composition, or a cosmetic composition.

5. A method of diagnosing stomach cancer or dementia, the method comprising the following steps:
(a) extracting DNAs from vesicles isolated from samples of a normal individual and a subj ect;
(b) performing PCR on the extracted DNA using a pair of primers prepared based on a gene sequence present in 16S rDNA to obtain each PCR product; and
(c) determining a case in which a content of vesicles derived from bacteria of the genus *Deinococcus* is lower than that of the normal individual sample, as stomach cancer or dementia, through quantitative analysis of the PCR product.

6. The method of claim 5, wherein the sample in Step (a) is blood.

## Patentansprüche

1. Zusammensetzung zur Verwendung in der Verhinderung, Behandlung oder Linderung von einer oder mehreren Krankheiten, ausgewählt aus der Gruppe bestehend aus einer Entzündungskrankheit und einer Kranialnervenkrankheit, umfassend von *Deinococcus radiodurans* abgeleitete Vesikel als Wirkstoff, und
wobei die Entzündungskrankheit eine durch Tumornekrosefaktor-a (TNF-α) vermittelte Krankheit ist, und
wobei die Kranialnervenkrankheit eines oder mehreres ist ausgewählt aus der Gruppe bestehend aus Demenz, Depression, Alzheimer-Krankheit und Autismus.

2. Zusammensetzung zur Verwendung nach Anspruch 1, wobei die Vesikel einen Durchschnittsdurchmesser von 10 bis 200 nm aufweisen.

3. Zusammensetzung zur Verwendung nach Anspruch 1 oder Anspruch 2, wobei die Vesikel natürlich oder künstlich von *Deinococcus radiodurans* abgesondert werden.

4. Zusammensetzung zur Verwendung nach einem der Ansprüche 1 bis 3, wobei die Zusammensetzung eine pharmazeutische Zusammensetzung, eine Nahrungsmittelzusammensetzung, eine Inhalationsmittelzusammensetzung oder eine kosmetische Zusammensetzung ist.

5. Verfahren zum Diagnostizieren von Magenkrebs, wobei das Verfahren folgende Schritte umfasst:
a) Extrahieren von DNA aus Vesikeln, die aus Proben eines unauffälligen Individuums und eines Subjekts isoliert wurden;
b) Durchführen von PCR an der extrahierten DNA unter Verwendung eines Primerpaars, das auf der Grundlage einer Gensequenz zubereitet wurde, die in 16S rDNA vorliegt, um jedes PCR-Produkt zu erhalten; und
c) Bestimmen eines Falls, in dem ein Inhalt von Vesikeln, die von Bakterien der Gattung *Deinococcus* abgeleitet sind, geringer ist, als der der normalen individuellen Probe, wie Magenkrebs oder Demenz, anhand qualitativer Analyse des PCR-Produkts.

6. Verfahren nach Anspruch 5, wobei die Probe in Schritt (a) Blut ist.

## Revendications

1. Composition destinée à être utilisée dans la prévention, le traitement ou l'atténuation d'une ou de plusieurs maladies sélectionnées dans le groupe constitué par une maladie inflammatoire et une maladie du nerf crânien, comprenant des vésicules dérivées de *Deinococcus radiodurans* en tant qu'ingrédient actif, et
dans laquelle la maladie inflammatoire est une maladie médiée par le facteur de nécrose tumorale alpha (TNF-α) et
dans laquelle la maladie du nerf crânien est une ou plusieurs maladies sélectionnées dans le groupe constitué par la démence, la dépression, la maladie d'Alzheimer et l'autisme.

2. Composition destinée à être utilisée selon la revendication 1, dans laquelle les vésicules présentent un diamètre moyen de 10 nm à 200 nm.

3. Composition destinée à être utilisée selon la revendication 1 ou la revendication 2, dans laquelle les vésicules sont sécrétées naturellement ou artificiellement à partir de *Deinococcus radiodurans.*

4. Composition destinée à être utilisée selon l'une quelconque des revendications 1 à 3, dans laquelle la composition est une composition pharmaceutique, une composition alimentaire, une composition d'inhalation ou une composition cosmétique.

5. Procédé de diagnostic du cancer de l'estomac ou de la démence, le procédé comprenant les étapes suivantes :
(a) l'extraction d'ADN de vésicules isolées à partir d'échantillons d'un individu normal et d'un sujet ;
b) la réalisation d'un PCR sur l'ADN extrait à l'aide d'une paire d'amorces préparées sur la base d'une séquence de gènes présente dans l'ADNr 16S pour obtenir chaque produit de PCR ; et
c) la détermination d'un cas dans lequel une teneur de vésicules dérivées de bactéries du gène *Deinococcus* est inférieure à celle de l'échantillon d'individu normal, en tant que cancer de l'estomac ou démence, au moyen d'une analyse quantitative du produit de PCR.

6. Procédé selon la revendication 5, dans lequel l'échantillon à l'étape (a) est du sang.
